# EUROPEAN PATENT SPECIFICATION

(11) **EP 0 921 830 B1**
(45) Date of publication and mention of the grant of the patent: **04.09.2002**
(21) Application number: 97921776.7
(22) Date of filing: 25.04.1997
(51) Int. Cl.: A61M 15/00, A61J 1/03, B65D 75/34

(54) **INHALER**
INHALATOR
INHALATEUR

(30) Priority: 25.04.1996 DE 19616418; 02.05.1996 DE 19617555; 15.05.1996 DE 19619536
(43) Date of publication of application: 16.06.1999
(73) Proprietor: AstraZeneca AB, 151 85 Södertälje (SE)
(72) Inventor: Von Schuckmann, Alfred, 47627 Kevelaer (DE)
(74) Representative: Shackleton, Nicola
(86) International application number: PCT/EP97/02144
(87) International publication number: WO 97/040876

(56) References cited:
- EP-A- 0 129 985
- EP-A- 0 189 276
- EP-A- 0 393 942
- EP-A- 0 511 726
- EP-A- 0 525 720
- WO-A-96/09085
- DE-A- 19 500 764
- US-A- 2 549 303
- US-A- 4 064 878
- US-A- 5 181 189
- US-A- 5 229 164
- US-A- 5 415 162
- US-A- 5 457 895

## Description

The present invention relates to an inhaler, more particularly an inhaler for administering dry powder, a support unit for an inhaler and a suction tube for an inhaler.

It is known in the treatment of respiratory conditions such as asthma to provide certain medicaments in the form of dry powder for inhalation.

It is also known to provide individual doses of such powders in the blisters of a blister pack. With such blister packs, however, either the user has manually to peel away the covering film from an individual blister to access the dose of powder or a complex device is required for rupturing the covering film or the cup-shaped portion of an individual blister. Where the user has manually to peel away the covering film, the task of finding the edge of the covering film and peeling the same away without spilling any of the powder contained therein can be difficult for some patients, particularly the young, the elderly or those actually experiencing an asthma attack. On the other hand, known devices for automatically rupturing a blister are complex and therefore costly to produce. Indeed, such known devices for rupturing the covering film or the cup-shaped portion of an individual blister can lead to parts of the covering film or the cup-shaped portion becoming detached, with the associated risk of those parts being inhaled. Furthermore, it has proved extremely difficult to rupture the covering film or the cup-shaped portion of a blister in such a way as to ensure that all of the powder is removed from the blister or at least that a consistent proportion of the powder is removed.

US-A-2549303 discloses an inhaler for administering dry powder containing medicament. The inhaler comprises a cartridge which includes a compartment which has a rupturable wall and contains dry powder containing medicament and a suction tube one end of which includes a frusto-conical extension for rupturing the rupturable wall of the cartridge and the other end of which is adapted for insertion into a respiratory orifice through which a user inhales.

WO-A-96/09085 discloses a device comprising the features defined in the pre-characterising portion of Claim 1 for aerosolizing dry powder containing medicament contained in a blister. The device comprises a dispersion chamber from which dry powder when dispersed is inhaled by a user, a feed tube assembly one end of which is in fluid communication with the dispersion chamber and the other end of which includes a piercing mechanism for rupturing the covering film of a blister, a feed tube through which powder is drawn from the blister and at least one air flow channel through which fluidizing air is drawn into the blister, and a pressurized gas source for delivering a high pressure gas to the feed tube assembly by means of which the powder in the blister is drawn into the dispersion chamber. The feed tube assembly is configured such that the high pressure gas draws fluidizing air through the at least one air flow channel into the blister where the powder contained therein is entrained and then through the feed tube into the dispersion chamber.

It is an aim of the present invention to provide an improved inhaler

Thus, the present invention provides an inhaler which is relatively simple to construct, of relatively low cost to the user and simple to use, but yet extremely effective in removing powder from a blister.

Furthermore, since the suction tube is a component separate from the blister pack, advantageously the suction tube can be easily cleaned as and when required. Indeed, the suction tube can be cleaned in any way, including total immersion in water, without there being any risk of contaminating or interfering with the operation of the inhaler. This is in contrast with known devices for automatically rupturing the covering film of a blister, where, even if the powder for administration is sealed in blisters, residual powder which has accumulated in other, particularly more complicated, parts of the device may not be fully removed and may interfere with the correct operation of the device after being dampened through a washing operation.

In one embodiment the support unit is a container within which the blister pack is in use housed.

Preferably, the support unit includes a support member including a plurality of cavities for receiving respective blisters of the blister pack, the cavities having the same arrangement as the apertures in the guide wall such that in use the blisters of the blister pack oppose the respective apertures.

More preferably, the support member is adapted to be slideable relative to the guide wall so as to allow loading and unloading of blister packs.

In another embodiment the support unit includes holding means for holding the substantially planar surface of the blister pack adjacent-the guide wall.

Preferably, the holding means comprises C-shaped lips for slideably receiving the blister pack.

Preferably, the apertures are defined by elongate sections.

More preferably, the length of the elongate sections is greater than the width of the blisters of the blister pack.

Preferably, the support unit includes one or more projections or recesses of predetermined size and position such that the support unit can only receive blister packs with corresponding recesses or projections.

More preferably, the one or more projections or recesses are sized and positioned such that the support unit can only receive blister packs in one relative orientation.

Preferably, the inlet section of the suction tube and each of the apertures are configured such that the inlet section of the suction tube can only be inserted through the apertures in one relative orientation.

More preferably, the cross-section of the inlet section of the suction tube and the cross-section of each of the apertures is rotationally asymmetric.

Yet more preferably, each of the apertures is generally circular in shape with at least one radial extension.

Still more preferably, a plurality of the apertures are linked by at least one radial extension.

Preferably, the support unit includes a holder for holding the suction tube when not in use.

More preferably, the holder comprises a chamber with a hinged lid.

Preferably, the inlet section of the suction tube includes a cutter having a cutting edge for cutting a covering film of a blister of the blister pack.

By providing the suction tube with a cutter the covering film of a blister is only cut when the suction tube is inserted into the blister. In this way, spillage as may occur when handling a blister pack with an open blister containing powder can be avoided.

More preferably, the cutter is located around only a part of the inlet of the suction'tube such that upon insertion of the inlet section of the suction tube into a blister of the blister pack the cutter cuts the covering film of the blister such as to leave a single cut film flap.

In this way, the covering film of a blister is never cut free and thus cannot be inhaled by a user.

Yet more preferably, the cutting edge of the cutter is disposed at the outer peripheral surface of the inlet section of the suction tube such that when the inlet section of the suction tube is inserted into a blister of the blister pack the cutter first cuts the covering film of the blister and then the inner peripheral surface of the inlet section of the suction tube pushes the cut film flap into the blister.

Preferably, the cutter comprises a plurality of axially-extending blades, each having cutting edges, separated by axially-extending gaps.

Such a cutter arrangement ensures that an air flow path exists between a blister and the inlet of the suction tube since air can always flow through the axially-extending gaps. Furthermore, by virtue of the relationship between the blades and the gaps, the covering film will tear to bridge the gaps between the blades.

Preferably, the inlet section of the suction tube includes at least one passageway for providing fluid communication between a blister of the blister pack and a space above the blister when the inlet section of the suction tube is inserted in the blister.

In this way, powder may be assuredly drawn from a blister since an air flow path is provided to the blister. In particular, by providing air flow paths at the outer periphery of a blister powder is more completely removed from the blister.

In one embodiment the at least one passageway comprises an open channel in the outer peripheral surface of the inlet section of the suction tube, the at least one passageway having one end located at a position along the length of the inlet section of the suction tube and the other end located adjacent the inlet of the suction tube.

Preferably, the other end of the at least one passageway is located adjacent the cutter.

In one preferred embodiment the other end of the at least one passageway is located at one of the gaps.

In another preferred embodiment the other end of the at least one passageway is located between adjacent gaps.

In another embodiment the at least one passageway comprises a conduit in the inlet section of the suction tube, the at least one passageway having an inlet located at a position along the length of the inlet section of the suction tube and an outlet located adjacent the inlet of the suction tube.

Preferably, the outlet of the at least one passageway is located adjacent the cutter.

In one preferred embodiment the outlet of the at least one passageway is located at one of the gaps.

In another preferred embodiment the outlet of the at least one passageway is located between adjacent gaps.

Preferably, the suction tube includes at least one passageway providing fluid communication between the inhalation channel and the atmosphere through which supplementary air is in use drawn into the inhalation channel on inhalation by the user.

Preferably, the inlet section of the suction tube includes a radially-extending member defining a shoulder at a predetermined axial distance form the distal end thereof, the shoulder being configured so as in use to abut the blister pack and prevent the inlet section of the suction tube from being inserted too far into a blister of the blister pack.

Preferably, the inhaler further comprises a member connecting the suction tube to the support unit in order to prevent the suction tube and the support unit from being separated from one another.

Medicaments suitable for administration by the present invention are any which may be delivered by inhalation and include for example β2-adrenoreceptor agonists, for example, salbutamol, terbutaline, rimiterol, fenoterol, reproterol, adrenaline, pirbuterol, isoprenaline, orciprenaline, bitolterol, salmeterol, formoterol, clenbuterol, procaterol, broxaterol, picumeterol, TA-2005, mabuterol and the like, and their pharmacologically acceptable esters and salts; anticholinergic bronchodilators, for example, ipratropium bromide and the like; glucocorticosteroids, for example, beclomethasone, fluticasone, budesonide, tipredane, dexamethasone, betamethasone, fluocinolone, triamcinolone acetonide, mometasone and the like, and their pharmacologically acceptable esters and salts; antiallergic medicaments, for example, sodium cromoglycate and nedocromil sodium; expectorants; mucolytics; antihistamines; cyclooxygenase inhibitors; leukotriene synthesis inhibitors; leukotriene antagonists; phospholipase-A2 (PLA2) inhibitors; platelet aggregating factor (PAF) antagonists and prophylactics of asthma; antiarrhythmic medicaments; tranquilisers; cardiac glycosides; hormones; antihypertensive medicaments; antidiabetic medicaments; antiparasitic medicaments; anticancer medicaments; sedatives; analgesic medicaments; antibiotics; antirheumatic medicaments; immunotherapies; antifungal medicaments; antihypotension medicaments; vaccines; antiviral medicaments; proteins; polypeptides and peptides, for example, peptide hormones and growth factors; polypeptide vaccines; enzymes; endorphines; lipoproteins and polypeptides involved in the blood coagulation cascade; vitamins; and others, for example, cell surface receptor blockers, antioxidants, free radical scavengers and organic salts of N,N'-diacetylcystine.

Preferred embodiments of the present invention will now be described hereinbelow by way of example only with reference to the accompanying drawings, in which:
Figure 1 illustrates a perspective view of an inhaler in accordance with a first embodiment of the present invention;
Figures 2 to 4 illustrate respectively top, end and side views of the support unit of the inhaler of Figure 1;
Figures 5 and 6 illustrate in enlarged scale orthogonal side views of the suction tube of the inhaler of Figure 1;
Figure 7 illustrates in enlarged scale a horizontal sectional view (along section I-I in Figure 6) of the suction tube of the inhaler of Figure 1;
Figure 8 illustrates in enlarged scale a top view of the upper, mouthpiece end of the suction tube of the inhaler of Figure 1;
Figure 9 illustrates in enlarged scale a bottom view of the lower end of the suction tube of the inhaler of Figure 1;
Figure 10 illustrates in enlarged scale the view of Figure 9;
Figure 11 illustrates in enlarged scale a vertical sectional view (along section II-II in Figure 6) of the suction tube of the inhaler of Figure 1;
Figure 12 illustrates in enlarged scale a vertical sectional view (along section III-III in Figure 5) of the suction tube of the inhaler of Figure 1;
Figure 13 illustrates in enlarged scale a vertical sectional view (along section IV-IV in Figure 1) of the inhaler of Figure 1, illustrated in use with a blister pack contained in the support unit;
Figure 14 illustrates in enlarged scale a fragmentary vertical sectional view (along section IV-IV in Figure 1) of the inhaler of Figure 1, illustrated in use with a blister pack contained in the support unit;
Figure 15 illustrates in enlarged scale a fragmentary vertical sectional view (along section V-V in Figure 1) of the inhaler of Figure 1, illustrated in use with a blister pack contained in the support unit;
Figure 16 illustrates a fragmentary top view of a modified support unit for the inhaler of Figure 1;
Figure 17 illustrates in enlarged scale a bottom view of the lower end of a modified suction tube for the inhaler of Figure 1;
Figure 18 illustrates in enlarged scale a fragmentary vertical sectional view (along section VI-VI in Figure 17 and corresponding to section IV-IV in Figure 1) of the suction tube of Figure 17 and the support unit of the inhaler of Figure 1, illustrated in use with a blister pack contained in the support unit;
Figure 19 illustrates a blister pack for the inhaler of Figure 1;
Figure 20 illustrates a perspective view of an inhaler in accordance with a second embodiment of the present invention;
Figure 21 illustrates a perspective view of a support unit for the inhaler of Figure 20;
Figure 22 illustrates in enlarged scale a fragmentary vertical sectional view (along section VII-VII in Figure 21) of the support unit of Figure 21;
Figure 23 illustrates in enlarged scale a fragmentary top view of the support unit of Figure 21;
Figure 24 illustrates in enlarged scale a fragmentary top view of a modified support unit for the inhaler of Figure 20;
Figure 25 illustrates in enlarged scale a fragmentary vertical sectional view (along section VIII-VIII in Figure 24) of the support unit of Figure 24;
Figure 26 illustrates a top view of a further modified blister pack for the inhaler of Figure 1;
Figure 27 illustrates in enlarged scale a fragmentary top view of the blister pack of Figure 26;
Figure 28 illustrates in enlarged scale a fragmentary vertical sectional view (along section IX-IX in Figure 27) of a modified tray for the support unit of the inhaler of Figure 1, illustrated with the blister pack of Figure 26 located thereon; and
Figure 29 illustrates a top view of a yet further modified blister pack for the inhaler of Figure 1.

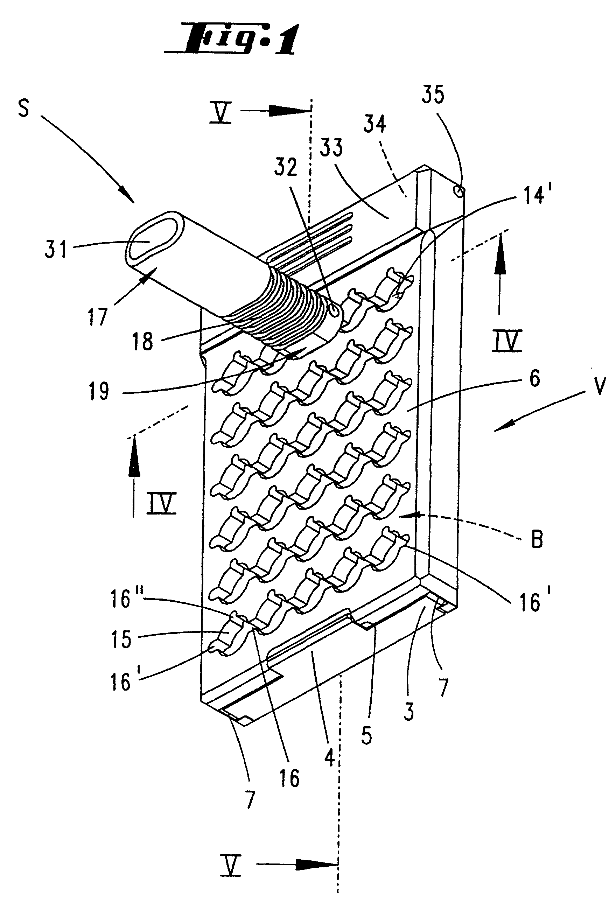

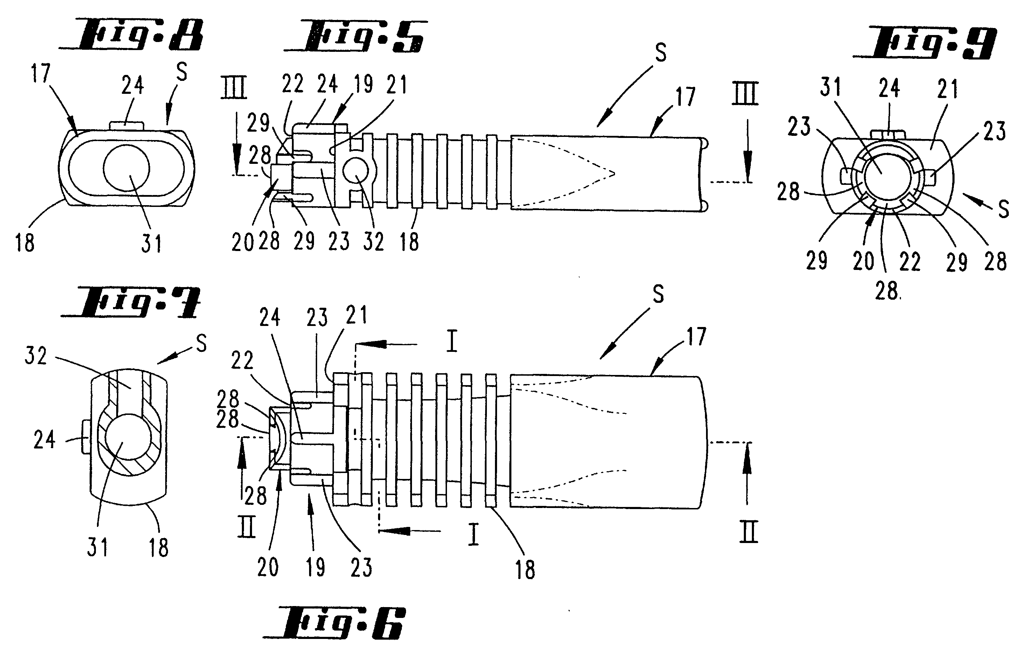

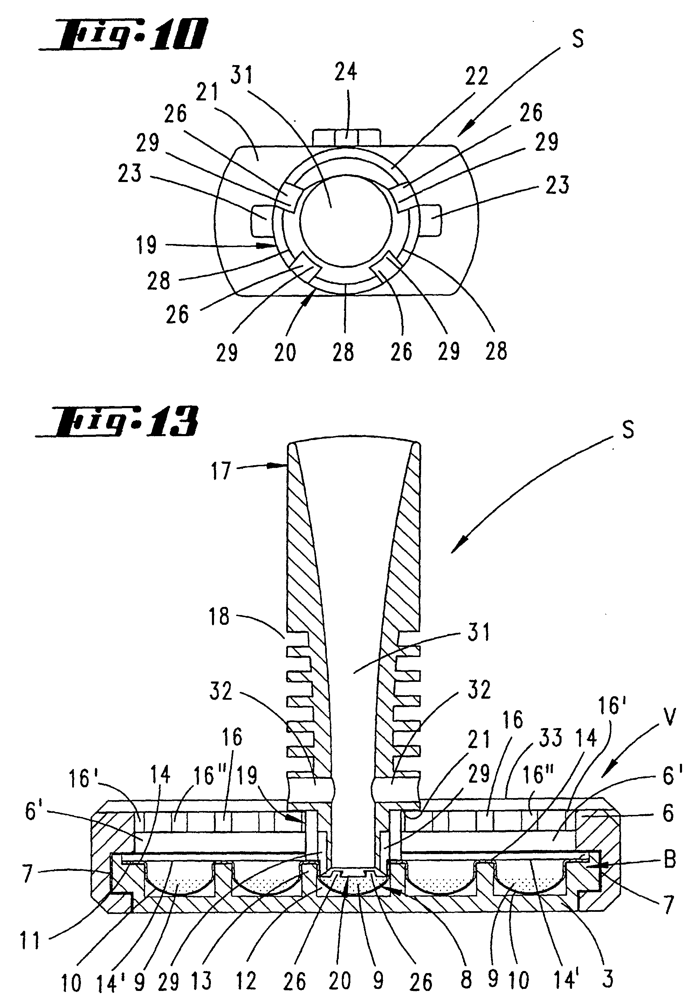

Figures 1 to 16 illustrate an inhaler or components thereof in accordance with a first embodiment of the present invention.

The inhaler comprises a support unit V for supporting a blister pack B and a suction tube S. In a preferred embodiment a protective cover may be provided for the support unit V.

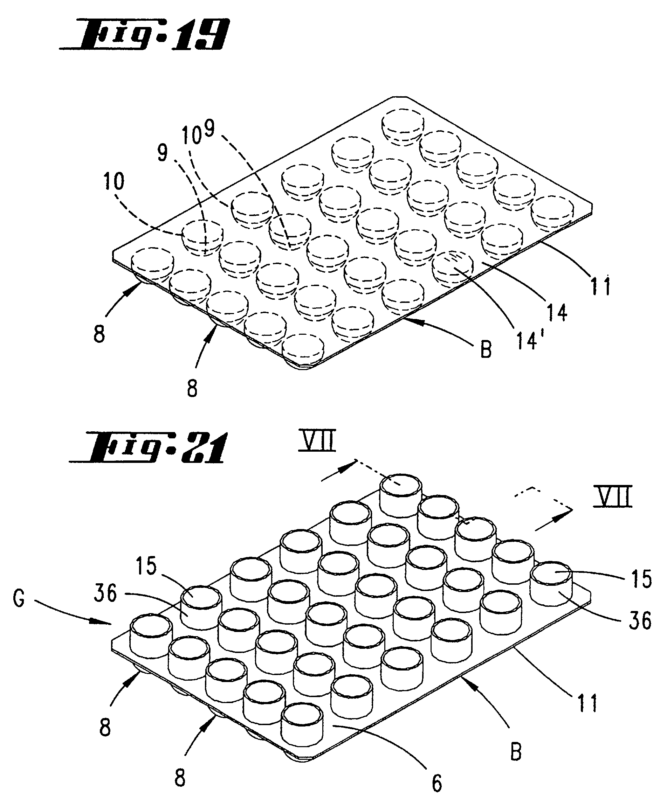

The support unit V, in this embodiment a container, houses a blister pack B, such as illustrated in Figure 19, the respective blisters 8 of which each contain a dose of powder containing medicament 9. The support unit V includes an array of apertures 15 which provide guide portions corresponding respectively to the array of blisters 8 in the blister pack B. The support unit V is typically of a size similar to a tissue or cigarette pack.

The suction tube S is preferably of a length which corresponds approximately to the small finger of an adult human hand. In use, one end section 19 providing an inlet, an inlet section, of the suction tube S is inserted into one of the apertures 15 and pushed down so as to rupture the covering film 14' of the blister 8 therebeneath.

As will be described hereinbelow, the suction tube S includes an inhalation channel 31 which extends between the inlet section 19 providing an inlet and an outlet section 17 providing an outlet and acting as a mouthpiece. Thus, with the inlet section 19 inserted into a blister 8, when a user inhales through the outlet section 17 the dose of powder containing medicament 9 in the blister 8 is drawn through the inhalation channel 31. As will be apparent, successive doses of powder containing medicament 9 are obtained by successively inserting the suction tube S into an aperture 15 corresponding to a previously unused blister 8.

In this embodiment the support unit V includes a chamber 34 in which the suction tube S is stored when not in use. The chamber 34 is closed by a lid 33 which is hinged about a pair of opposing hinge pins 35. The closing joint of the lid 33 is obliquely directed so as to maximise the surface area of the gripping portion on the lid 33. The closing joint is preferably at an angle of 45° with respect to the planar extent of the support unit V. Other arrangements are, of course, possible. For instance, the suction tube S could be inserted axially into an opening in the side of the support unit V. In any arrangement an elongate brush could be provided onto which the suction tube S is fitted. In this way, whenever the suction tube S is stored, the suction tube S is not only held in place by virtue of the brush extending through the inhalation channel 31, but is also cleaned. Indeed, as the suction tube S is pulled off the brush, the suction tube S is cleaned immediately prior to use.

In a preferred embodiment, in order to prevent the suction tube S and the support unit V from being separated and one being lost, the suction tube S can be attached to the support unit V by a member, such as a cord, particularly a very fine light cord-like member. In a particularly preferred embodiment the inhaler can include a sprung mechanism by means of which the member can be automatically retracted back into one or other of the suction tube S and the support unit V. For example, the member could be wound around a roller which unwinds against a relatively gentle resilient force.

The blister pack B to be used in the inhaler of this embodiment is preferably of the kind as illustrated in Figure 19. The blister pack B comprises a generally planar thin sheet 11 having a plurality of cup-shaped portions or depressions 10 formed therein each containing a dose of powder containing medicament 9. The blister pack B further comprises a thin film 14, such as a foil, preferably of aluminium, which covers the openings to the cup-shaped portions 10 and thereby seals the doses of powder containing medicament 9 in the cup-shaped portions 10 to provide blisters 8.

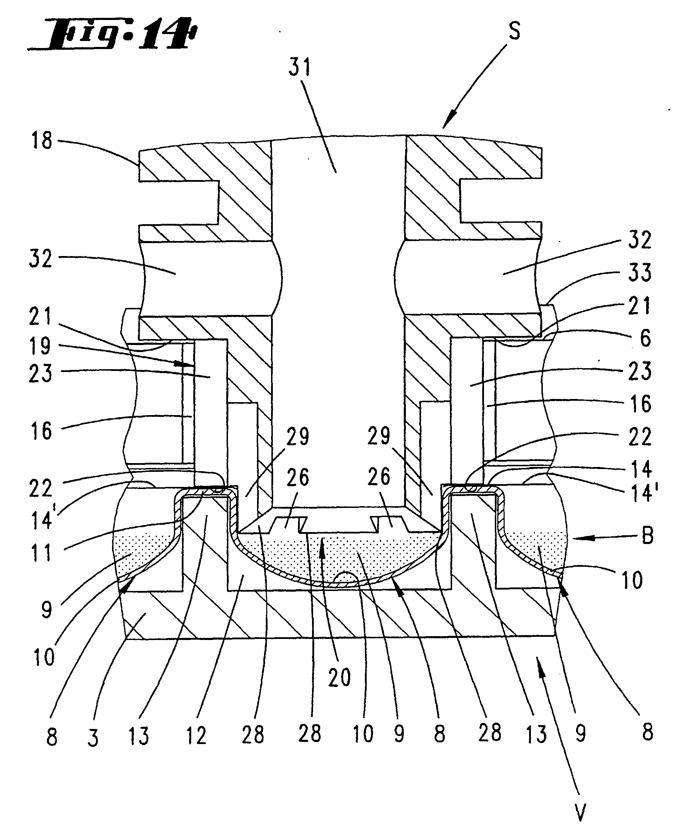

As described hereinabove, a blister pack B is located in the support unit V such that individual blisters 8 are positioned directly beneath respective apertures 15. In this embodiment the support unit V includes a substantially planar guide wall 6 against which the substantially planar surface of the blister pack B is disposed. The guide wall 6 includes the plurality of apertures 15 which provide the guide portions that guide the inlet section 19 of the suction tube S into respective blisters 8 in such a manner that the suction tube S cannot tilt relative to the support unit V. The apertures 15 may take the form of frames surrounding the blisters 8. In this embodiment, however, the under surface of the guide wall 6 is provided with elongate ribs 6', which, as illustrated in Figure 13, space the blister pack B from the under surface of the guide wall 6. The elongate ribs 6' are located between adjacent rows of apertures 15 and extend across the width of the support unit V. The elongate ribs 6' act to hold a blister pack B securely with respect to the guide wall 6 and a supporting tray 3 as will be described hereinbelow. By providing a space between the substantially planar surface of the blister pack B and the guide wall 6 a good flow of air around and into the cup-shaped portion 10 of any particular blister 8 is ensured.

As in this embodiment, the support unit V is preferably configured to allow the blister pack B to be replaced, such that one support unit V may be used with successive blister packs B as each is exhausted. It is possible to provide many different mechanisms for loading and unloading a blister pack B to and from the support unit V, such as by providing a hinged back part. In this embodiment the support unit V includes a slideable tray 3. The tray 3 is slideable in and out of one end of the support unit V and is preferably provided with a plurality of depressions or cavities 12 having the same arrangement as the blisters 8 in the blister pack B. In this way, the blister pack B may be laid onto the tray 3 when withdrawn and securely located thereto such that when the tray 3 is inserted back into the support unit V the blisters 8 are correctly aligned with the apertures 15 in the guide wall 6 of the support unit V. The cavities 12 correspond, at least in outline, to the cup-shaped portions 10 of the blisters 8 in the blister pack B, which, as illustrated, are cylindrical in the region adjacent the covering film 14'. The cavities 12 define a crater-like structure ordered in the form of criss-crossing rows, with partition walls 13 supporting the blister pack B. In this embodiment, in order to facilitate removal of the tray 3, the tray 3 is provided with a tab or handle 4 which protrudes into a recess 5 in the guide wall 6. As illustrated, the recess 5 is formed in one short side of the guide wall 6 without any overhang. In a preferred embodiment the tray 3 is provided with a catch to secure the same in the loaded position. The catch may be provided merely as a detent on the tray 3 or as part of the handle 4, such that the tray 3 is only released when the user deflects the handle 4 in some way. As illustrated, in order to enable the tray 3 to be slid in and out of the support unit V, the support unit V includes guides 7 having a generally C-shaped profile.

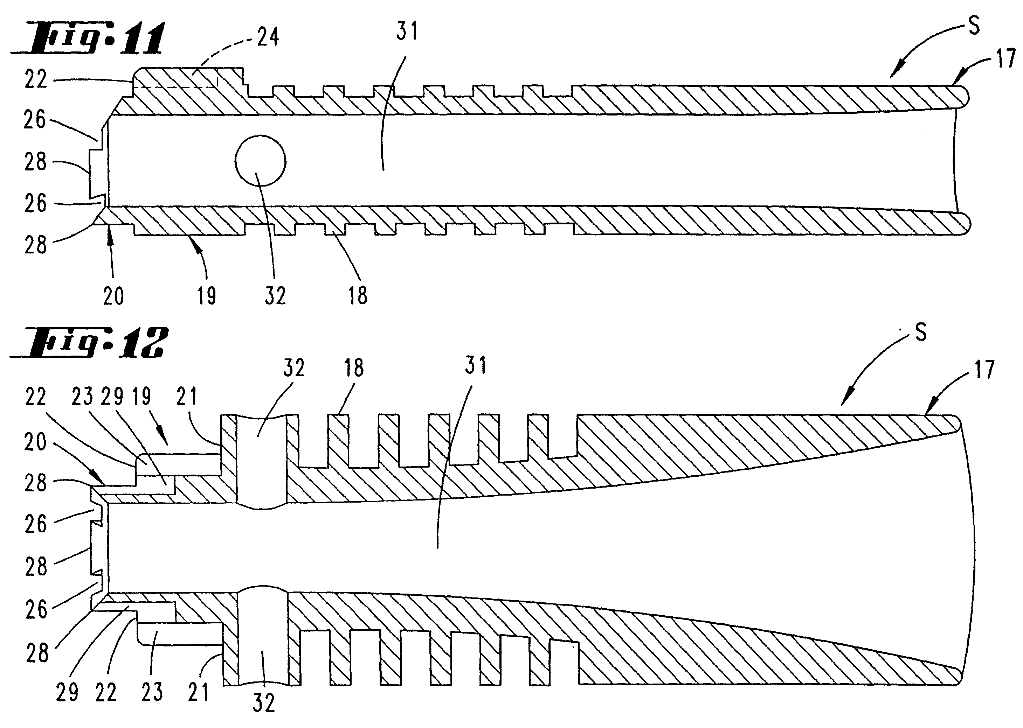

As described hereinabove and best illustrated by the cross-sections of Figures 11 and 12, the suction tube S generally comprises an elongate body having an inlet section 19 providing an inlet at one end, an outlet section 17 providing an outlet and acting as a mouthpiece at the other end and an inhalation channel 31 providing fluid communication between the inlet and the outlet. In this embodiment the outlet section 17 of the suction tube S is generally oval in shape, whereas the inlet section 19 is generally circular in shape.

As will become apparent, this configuration is not essential, but is advantageous. In particular, a flattened oval section is more comfortable in the mouth than a circular section. Furthermore, by shaping the inhalation channel 31 so as to flare outwardly from the inlet to the outlet, a higher velocity air flow is achieved at the inlet, thereby improving both the removal of the dose of powder containing medicament 9 from the blister 8 and the deagglomeration of that powder.

As illustrated, despite the inhalation channel 31 being tapered, the outer shape of the suction tube S is generally oval along most of the length thereof, at least up to a shoulder 21 which in use abuts the upper surface of the guide wall 6. The flats of the oval provide surfaces which can be gripped by the fingers of a user. In this embodiment, in order to maintain the oval shape, ribs 18 are provided down the length of the suction tube S. The provision of the ribs 18 is preferred in allowing for the suction tube S to be gripped securely by a user. A secure grip is necessary since some force has to be applied to the suction tube S on insertion into an aperture 15 in order to rupture the covering film 14' sealing the opening to the cup-shaped portion 10 of the respective blister 8.

In this embodiment, as illustrated, axially at the bottom of the plurality of ribs 18, and just axially above the shoulder 21, the suction tube S includes passageways 32 in fluid communication with the inhalation channel 31. The passageways 32, in this embodiment transversely directed to the longitudinal axis of the suction tube S, provide supplementary air flow paths which allow supplementary air to be drawn into the inhalation channel 31 and mix with the air and powder mixture drawn through the inhalation channel 31 from the blister 8. The provision of such supplementary passageways 32 provides that for each unit volume of air inhaled the user inhales a reduced amount powder containing medicament. Furthermore, the action of the supplementary air mixing with the air and powder mixture flowing through the inhalation channel 31 induces turbulence and assists in the deagglomeration of that powder. This effect is enhanced by the inhalation channel 31 being tapered as described hereinabove. Preferably, the cross-section of the passageways 32 together correspond approximately to the circular cross-section of the inhalation channel 31 in the inlet section 19 of the suction tube S.

As described hereinabove, the passageways 32 are located adjacent the shoulder 21. The location of the passageways 32 in this position is advantageous since, when the inlet section 19 of the suction tube S is inserted into an aperture 15, the presence of the adjacent guide wall 6 ensures that it is almost impossible for a user accidentally to block the supplementary passageways 32 with his or her fingers.

In this embodiment, as best illustrated in Figures 11 and 12. the inlet section 19 of the suction tube S includes a generally annular cutter 20. The inlet section 19 of the suction tube S is shaped to fit securely in the apertures 15, whilst allowing air to be drawn down around the outer peripheral surface thereof into the ruptured blister 8 below. In this embodiment the inlet section 19 of the suction tube S is matched cross-sectionally to the shape of the apertures 15 which are preferably circular such that an interruption free, well supporting contact is achieved with the guide wall 6. In this embodiment the inlet section 19 of the suction tube S is a plug connection having frictional contact with the apertures 15.

As described hereinabove, the suction tube S includes a shoulder 21 which, in use, rests in a tilt-preventing manner on the upper surface of the guide wall 6 so as to ensure that the inlet section 19 of the suction tube S is always inserted into each aperture 15 and associated blister 8 to the same extent. It will, of course, be appreciated that the inlet section 19 of the suction tube S is of such a length as to position the inlet of the suction tube S in that position in the cup-shaped portion 10 of a blister 8 most suited to withdrawing therefrom the dose of powder containing medicament 9.

In this embodiment'the inlet section 19 of the suction tube S includes passageways 29 formed as axially-directed channels in the outer peripheral surface thereof. As will become apparent hereinbelow and, indeed, as illustrated in Figure 14, the passageways 29 provide an air flow path from above the surface of the blister pack B to a position below the ruptured covering film 14' of a blister 8. In this way, during inhalation through the suction tube S, air is drawn down through the passageways 29 and into the cup-shaped portion 10 of the blister 8 so as to pick up the dose of powder containing medicament 9 contained therein. The resulting air and powder mixture containing medicament is then drawn through the inhalation channel 31 and into the lungs of a user.

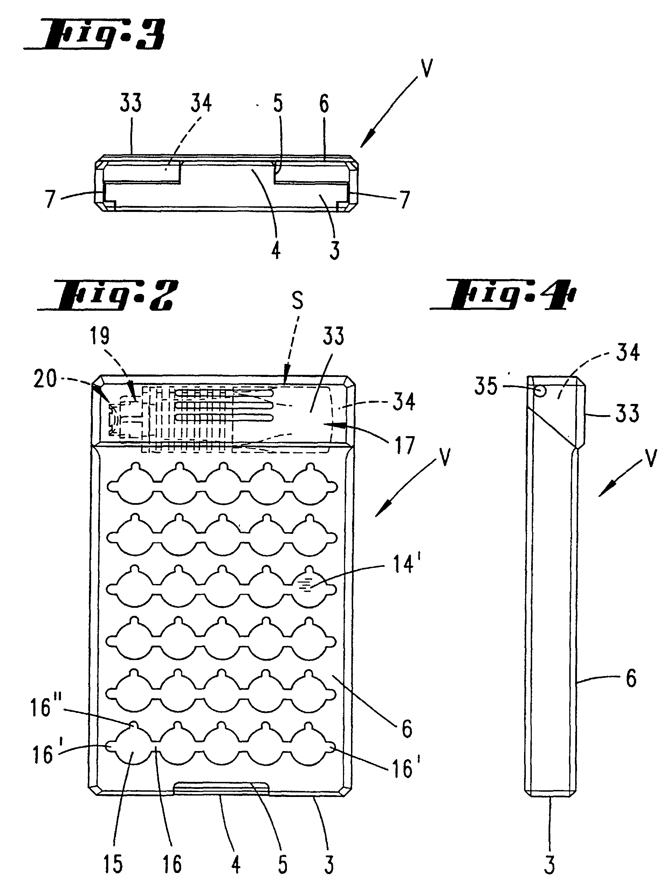

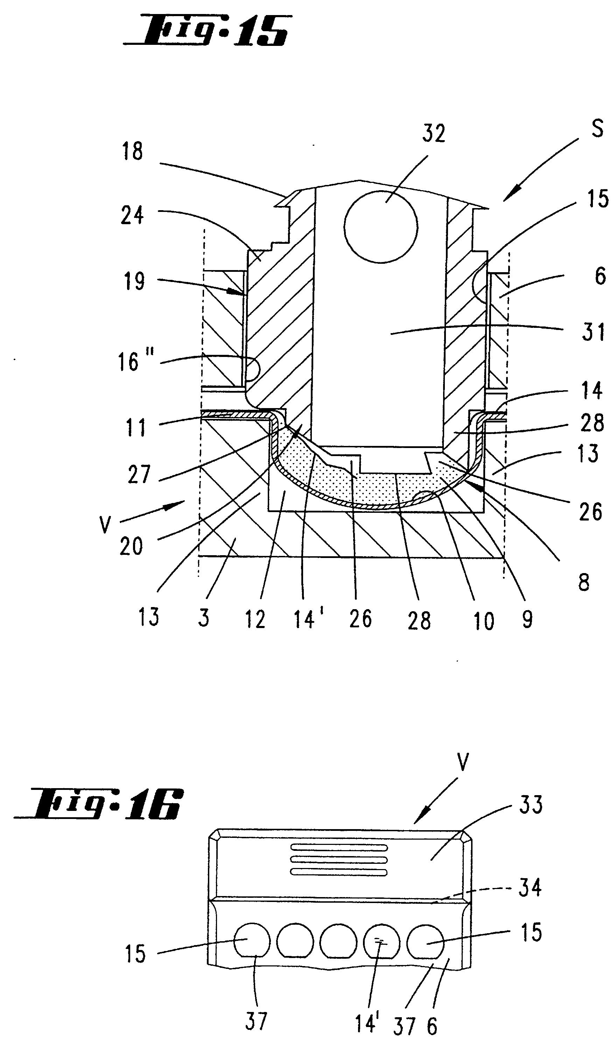

As illustrated, the cutter 20 includes a plurality of cutting blades 28 arranged around a greater part of the outer peripheral surface thereof. In this embodiment, where the cutter 20 is considered to comprise four sectors, the cutter 20 includes part-annular cutting blades 28 in three of the four sectors, with one sector not including a cutting blade 28. The sectors and hence cutting blades 28 are separated from one another by gaps 26. In this embodiment, as illustrated in Figures 5 and 15, the non-cutting sector of the cutter 20 tapers inwardly to the distal end of the cutter 20. Indeed, the cutter 20 may generally be angled relative to the longitudinal axis of the suction tube S such that the cutting blade 28 opposite the non-cutting sector contacts the covering film 14' of the respective blister 8 before the other cutting blades 28.

In use, when the suction tube S is inserted into one of the apertures 15, the cutting blades 28 of the cutter 20 come into contact with an unbroken covering film 14'. Upon inserting the suction tube S further into the aperture 15, the cutting blades 28 begin to cut the covering film 14'. It will, of course, be appreciated that the covering film 14' opposing the gaps 26 is not cut directly by one of the cutting blades 28, but rather, the gaps 26 are of such a size relative to the cutting blades 28 and the blister 8 that the covering film 14' tears so as to provide a continuous cut in the covering film 14' between the cutting blades 28. As . mentioned hereinabove, the cutting blades 28 of the cutter 20 do not extend around the entire annulus of the inlet section 19 of the suction tube S, but the cutter 20 includes a sector, preferably of about 90°, which does not cut the covering film 14'. In this embodiment the non-cutting sector of the cutter 20 does not contact the covering film 14' until the cutting blades 28 have cut the covering film 14' and are located in the cup-shaped portion 10 of the blister 8. The cutting blades 28 are sharpened cutting edges provided by the full radial thickness of the cutter 20, with the cutting edges being disposed at the outer radial surface of the radial thickness of the cutter 20. Therefore, as the inlet section 19 of the suction tube S is pushed further into the blister 8, the cut covering film 14' is pushed downwards by the cutter 20. That single section of the covering film 14' which is not cut thus acts as a hinge 27. With the inlet section 19 of the suction tube S fully inserted into the blister 8 the non-cutting sector of the cutter 20 contacts the covering film 14', but only to ensure that the cut covering film 14' is pushed down into the cup-shaped portion 10 of the blister 8, thereby ensuring that the inlet of the suction tube S defined by the cutter 20 is open to the interior of the blister 8.

In a preferred embodiment the covering film 14' is cut so as to leave the hinge 27 at an upper side as viewed in Figure 1. In normal use, the support unit V will be held as illustrated in Figure 1, whereby the doses of powder containing medicament 9 in the individual blisters 8 will collect away from the hinge 27 allowing unimpeded withdrawal.

It will be appreciated that the above-described cutting and insertion arrangement ensures not only reliable and consistent cutting of the covering films 14', but also that a cut covering film 14' is retained by the hinge 27 and cannot be inhaled by a user. In addition, the passageways 29 in the outer radial surface of the inlet section 19 of the suction tube S cannot be blocked by the covering film 14' and ensure that air is effectively drawn into the blister 8 under the covering film 14'. The passageways 29 also direct air into the outer periphery of the cup-shaped portion 10 of the blister 8 at a plurality of positions so as to ensure that all of the dose of powder containing medicament 9 in the blister 8 is exposed to a flow of air. Furthermore, the gaps 26 between the cutting blades 28 ensure that the resulting air and powder mixture is always able to flow past the cut covering film 14' and into the inhalation channel 31.

Referring to Figure 2, it will be noted that the apertures 15 in the guide wall 6 of the support unit V include radial extensions 16, 16', 16''. Indeed, in order to use the space available most efficiently, horizontally adjacent apertures 15 are linked by a common extension 16. Corresponding to the extensions 16, 16', 16'' the inlet section 19 of the suction tube S is provided with protrusions 23, 24. The corresponding extensions 16, 16', 16'' and protrusions 23, 24 fix the suction tube S more securely in the apertures 15 and therefore make the inhaler easier and more pleasing to use. The use of an asymmetric arrangement of extensions 16, 16', 16'' and protrusions 23, 24 means that the suction tube S may only be inserted in one particular predetermined orientation. This provides more consistent and pleasing operation for the user. Furthermore, if the cup-shaped portions 10 of the blisters 8 have an asymmetric shape, the suction tube S is advantageously always inserted with the correct orientation.

Irrespective of the nature of the blisters 8, ensuring that the suction tube S can only be inserted into each aperture 15 with one orientation has the advantage that the user is only able to insert the suction tube S into a previously used blister with the same orientation as was previously used. If a user were accidentally to be able to insert the suction tube S into a previously used blister with another orientation to that used previously, then the cut covering film 14' could be cut free, with the risk that the user could inhale the same. However, with the preferred arrangement described above, this is not possible.

As illustrated in Figure 14, the protrusions 23, 24 abut the top surface of the blister pack B. Indeed, where provided, the protrusions 23, 24 define a shoulder 22 that in use abuts the blister pack B. The abutment of the shoulder 22 fixes the blister pack B against the upper side of the partition walls 13 circumscribing the respective cavity 12. In this way, it is ensured that, irrespective of the position of the shoulder 22 because of variations occurring for instance for tolerance reasons, the cutter 20 is not inserted too far into a blister 8. This is particularly important, since, if the inlet section 19 of the suction tube S were inserted too far, the non-cutting sector of the cutter 20 could tear the covering film 14', causing the covering film 14' to be cut free.

Of course, it will be appreciated that it is possible to use many other different shapes for fixing the orientation of the suction tube S relative to the support unit V. Figure 16 illustrates one possibility, namely the use of apertures 15 with flattened sides 37. Preferably, the flattened sides 37 lie diametrically opposite the non-cutting sector of the cutter 20. This configuration requires an even shorter length of cut and the opening at the end face of the inlet section 19 of the suction tube S is thus kept even more free. Moreover, due to gravity, the dose of powder containing medicament 9 accumulates in front of the free end of the covering film 14'.

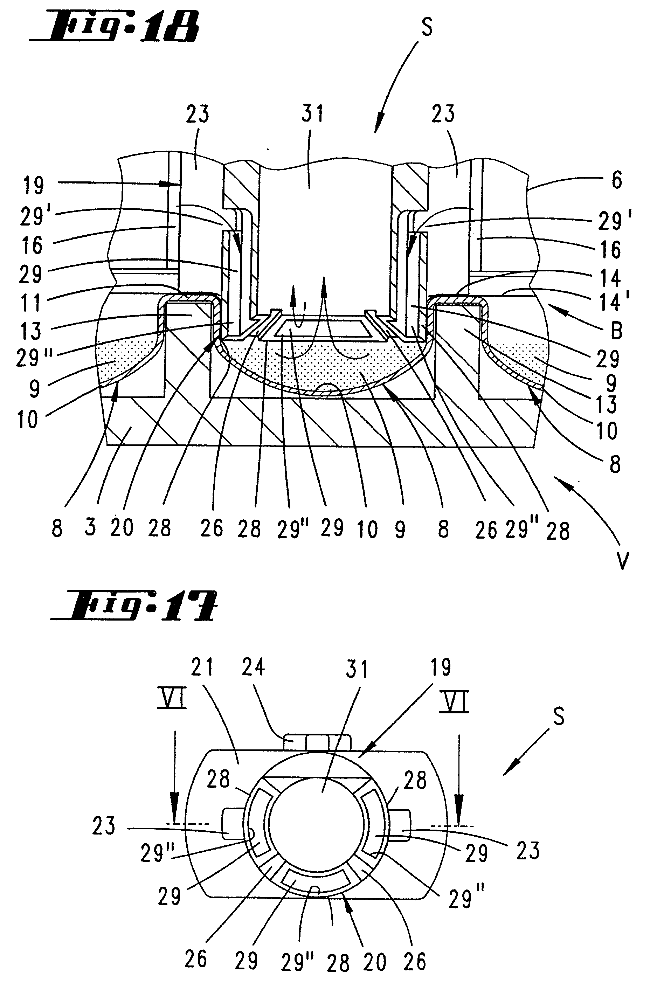

Figures 17 and 18 illustrate a modified suction tube S for the inhaler of the first embodiment. In this suction tube S the passageways 29 are not channels in the outer peripheral surface of the inlet section 19 of the suction tube S, but are conduits within the tubular member defining the inlet section 19 of the suction tube S having broad sector-shaped outlets 29'' adjacent the cutting blades 28 and between the gaps 26 and inlets 29' in the outer peripheral surface of the inlet section 19. This modified suction tube S advantageously provides a flow of air into the blister 8 around a greater length of the periphery thereof.

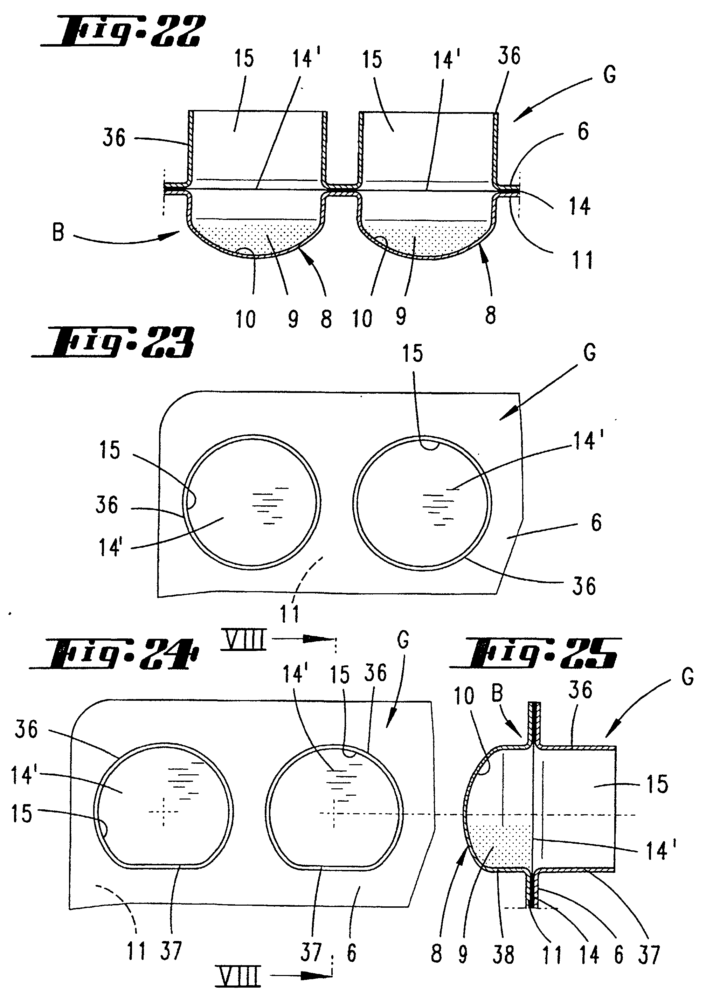

Figures 20 to 25 illustrate an inhaler or components thereof in accordance with a second embodiment of the present invention.

The inhaler comprises a support unit G and a suction tube S. The suction tube S is of the same kind as described hereinabove in relation to the inhaler of the first embodiment. The support unit G comprises a guide wall 6 of essentially the same kind as described hereinabove in relation to the inhaler of the first embodiment, with stubs 36 defining the apertures 15 that act as guide portions. The stubs 36 receive the inlet section 19 of the suction tube S such that the suction tube S is not able to tilt. In a preferred embodiment the support unit G is formed of a plastics material.

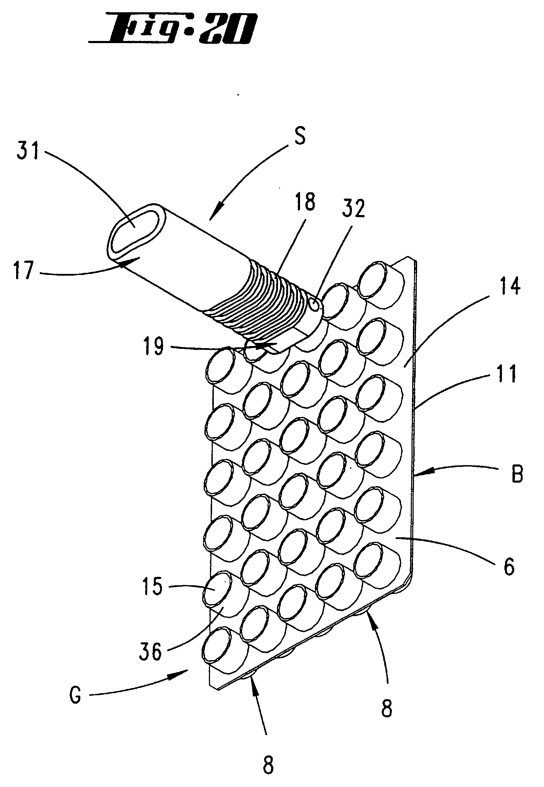

In a first variant, as illustrated in Figure 21, the support unit G is an integral part of a blister pack. The guide wall 6 of the support unit G is fixed, for instance, by adhesive or heat sealing to the covering film 14 of a blister pack B of the same kind as described hereinabove in relation to the inhaler of the first embodiment.

In a second variant the support unit G is adapted for use with successive blister packs B of the same kind as described hereinabove in relation to the inhaler of the first embodiment. This variant of the support unit G includes means for releasably securing a blister pack B to the under surface of the guide wall 6 thereof and holding the same in the correct relative position. In a preferred embodiment three of the four edges of the guide wall 6 could be provided with generally C-shaped lips, such that a blister pack B could be slid into position from that edge without such a lip.

Functionally, the support unit G of this embodiment is used in the same way as the support unit V described hereinabove in relation to the inhaler of the first embodiment. In particular, the inlet section 19 of the suction tube S which includes the cutter 20 is inserted into a respective aperture 15 so as to rupture the respective covering film 14'.

In a preferred embodiment the support unit G includes means for attaching the suction tube S thereto, for example, one or more clips such as resilient claws.

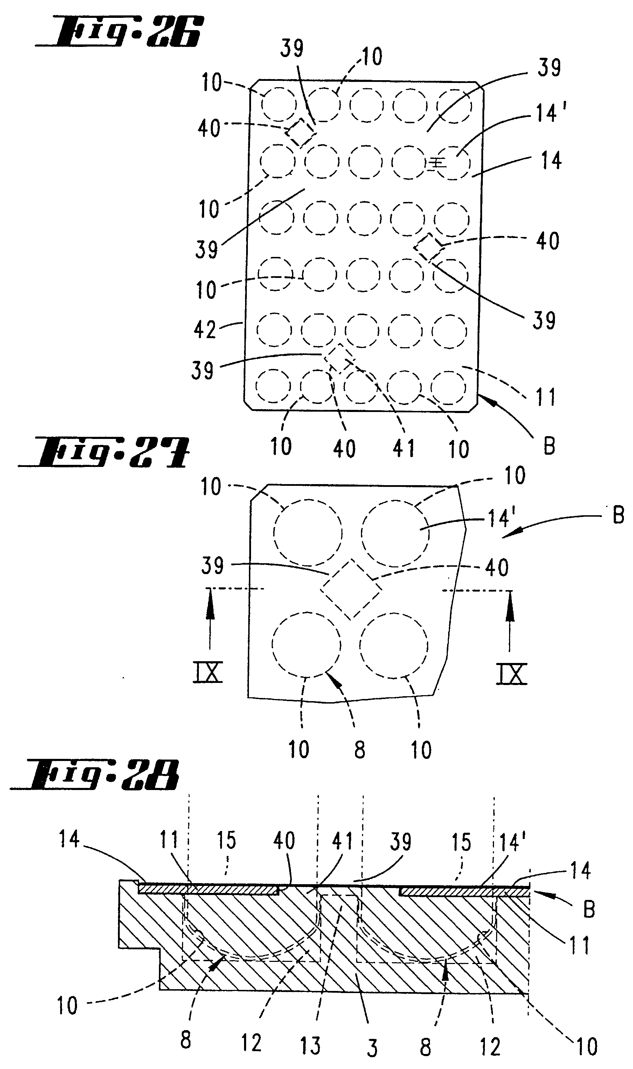

In the variant of the support unit G as illustrated in Figures 21 to 23 neither the stubs 36 of the guide wall 6 nor the inlet section 19 of the suction tube S are configured so as to ensure that the suction tube S can only be inserted in one relative orientation into a respective stub 36. However, it will, of course, be appreciated that the stubs 36 and the inlet section 19 of the suction tube S can be so configured. In a preferred embodiment, as illustrated in Figures 24 and 25 and similarly to the modified support unit V as illustrated in Figure 16, the apertures 15 of the stubs 36 may each be provided with a flattened portion 37 to ensure that the suction tube S is always inserted into an aperture 15 with the same relative orientation.

In the support units G of Figures 22 and 23 and Figures 24 and 25 the stubs 36 have the same cross-section as the upper sections of the cup-shaped portions 10 of the blisters 8. In particular, the blisters 8 of the support unit G of Figures 24 and 25 each have a flattened portion 38 which matches the flattened portion 37 of each stub 36. However, it is not necessary for the stubs 36 to be of the same shape as the cup-shaped portions 10 of the blisters 8. Desirably, that part of inlet section 19 of the suction tube S which is inserted into a blister 8 should encompass substantially all of the area of the opening of the blister 8 so as to promote the withdrawal of the dose of powder containing medicament 9 from the blister 8. However, other portions of the suction tube S may extend radially beyond the opening of the blister 8. In this way, the stubs 36 can be of any shape or size. Indeed, if at least part of the stubs 36 has a radial dimension greater than that of the blisters 8, the suction tube S may include a shoulder such as shoulder 22 described hereinabove in relation to the inhaler of the first embodiment.

In the preferred inhalers of the above-described first and second embodiments the inlet sections 19 of the suction tubes S and the apertures 15 in the guide walls 6 of the support units V, G are configured so as to ensure that the suction tubes S can only be inserted through respective apertures 15 in one relative orientation. It is, however, further preferred that the inlet sections 19 of the suction tubes S and the apertures 15 in the support units V, G be shaped and/or sized according to the medicament of use so as to avoid the possibility of cross-contamination of medicaments. It is envisaged that cross-contamination of medicaments could possibly occur where a user has to take two or more different medicaments, but by shaping and/or sizing the suction tubes S and the apertures 15 in the support units V, G uniquely according to the medicament the possibility of the user inadvertently using the wrong suction tube S is obviated.

Furthermore, where the support units V, G are reusable, it is important that blister packs B are loaded into support units V, G with the same orientation and that any loaded blister pack B contains the correct medicament. It is, therefore, preferred that the blister packs B include a physical feature representative of the orientation and/or the contained medicament. In an electronically-controlled inhaler the blister packs B could be provided with electronically-readable information, such as a magnetic strip or optically-readable bar coding. However, in relatively simple inhalers of the kind as described hereinabove it is proposed to provide the blister packs B with features having particular shapes, sizes and positions representative of the orientation and/or the contained medicament. In particular, in order to ensure that blister packs B are loaded into support units V, G with the correct orientation, the features are asymmetrically disposed with respect to the possible orientations for insertion.

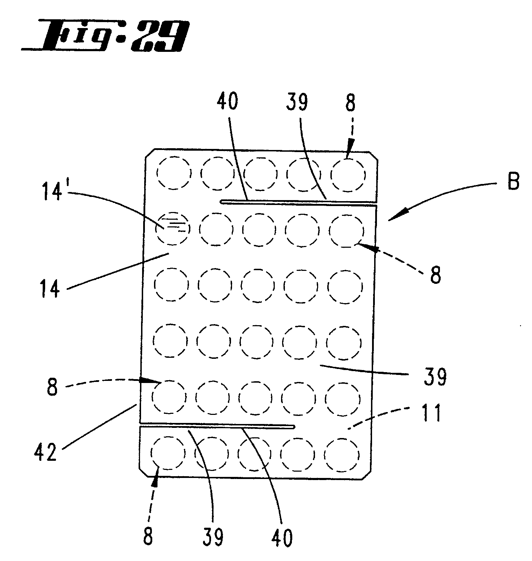

A blister pack B modified to include features representative of the orientation and/or the contained medicament is illustrated in Figures 26 and 27. This modified blister pack B includes first, second and third openings 40 in the sheet 11 in which the cup-shaped portions 10 of the blisters 8 are formed. The openings 40 are located in the intermediate zones 39 between the cup-shaped portions 10 defining the blisters 8. In the illustrated embodiment the openings 40 are hidden by the covering film 14. In a preferred embodiment the openings 40 are located at the corners of a square or rectangle. This modified blister pack B is particularly suited to the inhaler of the first embodiment where the blister pack B is laid onto a tray 3 which is then slid into a support unit V. A tray 3 modified to receive such a blister pack B is illustrated in Figure 28. The tray 3 includes protrusions 41 corresponding in size, shape and position to the openings 40 in the blister pack B. In this way, when the blister pack B is laid onto the tray 3 in the correct orientation the protrusions 41 fit into the openings 40. As will be apparent from Figure 26, if the blister pack B were to be laid onto the tray 3 with the opposite orientation, the protrusions 41 on the tray 3 would not align with the openings 40 in the blister pack B, such that the blister pack B would stand proud of the tray 3 and prevent the tray 3 from being slid into the support unit V. The asymmetric arrangement of the openings 40 and the protrusions 41 ensures that the blister pack B cannot be loaded into the support unit V with the incorrect orientation. It will further be appreciated that by selecting a different asymmetric arrangement of openings 40 and protrusions 41 for each different medicament, then blister packs B containing only the correct medicament can be loaded into the support unit V. In the illustrated embodiment the blister pack B includes twenty intermediate zones 39 in which openings 40 could be formed, thus giving scope for many possible combinations.

In the above-described modified blister pack B the openings 40 and the projections 41 have a lozenge-shaped or square outline. Other configurations are, of course, possible. One such further blister pack B is illustrated in Figure 29. This further modified blister pack B includes first and second slits 40 that extend inwardly from the peripheral edge 42. In this modified blister pack B the first and second slits 40 are paracentral and extend oppositely in a direction parallel to the narrow sides and over a length greater than half of the length of the narrow sides. In this way, the slits 40 overlap each other in the central region of the blister pack B. For such a modified blister pack B the tray 3 of the support unit V would, for example, include web or blade like protrusions.

In a yet further modified blister pack B the blisters 8 could be shaped and/or positioned to identify the orientation and/or the contained medicament. Alternatively, the blister pack B could include small appropriately-positioned protrusions which could be formed in the same thermoforming step as the cup-shaped portions 10 of the blisters 8.

For the above-described variant of the support unit G of the inhaler of the second embodiment which is reusable, protrusions and/or recesses can be provided on the upper substantially planar surface of the blister pack B and the under surface of the guide wall 6 of the support unit G which opposes the same. Specifically, in the preferred embodiment of the support unit G which includes C-shaped lips on opposing edges for receiving a blister pack B, the support unit G could be tapered such that only correspondingly-tapered blister packs B could be received in the one orientation.

## Claims

1. An inhaler for administering dry powder, in combination with a blister pack (B), the inhaler comprising a suction tube (S) having an inlet at one end and an outlet at the other end, the suction tube (S) including at the one end an inlet section (19) configured for insertion into the blister pack (B) having a plurality of blisters (8) each containing a dose of powder containing medicament (9) and an inhalation channel (31) providing fluid communication between the inlet and the outlet through which powder is, in use, drawn on inhalation by a user **characterised in that** the inhaler further comprises a support unit (V,G) configured to support the blister pack (B), the support unit (V,G) including a guide wall (6) adjacent which the blister pack (B) is, in use, disposed, the guide wall (6) including a plurality of apertures (15) acting as guide portions in alignment with respective blisters (8) of the blister pack (B), each aperture (15) being for guiding the inlet section (19) of the suction tube (S) into a respective blister (8) of the blister pack (B) and supporting the suction tube (S) when so guided.

2. The inhaler according to Claim 1, wherein the support unit (V) is a container within which the blister pack (B) is in use housed.

3. The inhaler according to Claim 2, wherein the support unit (V) includes a support member (3) including a plurality of cavities (12) for receiving respective blisters (8) of the blister pack (B), the cavities (12) having the same arrangement as the apertures (15) in the guide wall (6) such that in use the blisters (8) of the blister pack (B) oppose the respective apertures (15).

4. The inhaler according to Claim 3, wherein the support member (3) is adapted to be slidable relative to the guide wall (6) so as to allow loading and unloading of blister packs (B).

5. The inhaler according to Claim 1, wherein the support unit (G) includes holding means for holding the substantially planar surface of the blister pack (B) adjacent the guide wall (6).

6. The inhaler according to Claim 5, wherein the holding means comprises C-shaped lips for slidably receiving the blister pack (B).

7. The inhaler according to Claim 5 or 6, wherein the apertures (15) are defined by elongate sections (36).

8. The inhaler according to Claim 7, wherein the length of the elongate sections (36) is greater than the width of the blisters (8) of the blister pack (B).

9. The Inhaler according to any of Claims 1 to 8, wherein the support unit (V,G) includes one or more projections or recesses (41) of predetermined size and position such that the support unit (V.G) can only receive blister packs (B) with corresponding recesses or projections (40).

10. The inhaler according to Claim 9, wherein the one or more projections or recesses (41) are sized and positioned such that the support unit (V,G) can only receive blister packs (B) in one relative orientation.

11. The inhaler according to any of Claims 1 to 10, wherein the inlet section (19) of the suction tube (S) and each of the apertures (15) are configured such that the inlet section (19) of the suction tube (S) can only be inserted through the apertures (15) in one relative orientation.

12. The inhaler according to Claim 11, wherein the cross-section of the inlet section (19) of the suction tube (S) and the cross-section of each of the apertures (15) is rotationally asymmetric.

13. The inhaler according to Claim 12, wherein each of the apertures (15) is generally circular in shape with at least one radial extension (16,16',16").

14. The inhaler according to Claim 13, wherein a plurality of the apertures (15) are linked by at least one radial extension (16).

15. The inhaler according to any of Claims 1 to 14, where the support unit (V,G) includes a holder for holding the suction tube (S) when not in use.

16. The inhaler according to Claim 15, wherein the holder comprises a chamber (34) with a hinged lid (33).

17. The inhaler according to any of Claims 1 to 16, wherein the inlet section (19) of the suction tube (S) includes a cutter (20) having a cutting edge for cutting a covering film (14') of a blister (8) of the blister pack (B).

18. The inhaler according to Claim 17, wherein the cutter (20) is located around only a part of the inlet of the suction tube (S) such that upon insertion of the inlet section (19) of the suction tube (S) into a blister (8) of the blister pack (B) the cutter (20) cuts the covering film (14') of the blister (8) such as to leave a single cut film flap.

19. The inhaler according to Claim 18, wherein the cutting edge of the cutter (20) is disposed at the outer peripheral surface of the inlet section (19) of the suction tube (S) such that when the inlet section (19) of the suction tube (S) is inserted into a blister (8) of the blister pack (B) the cutter (20) first cuts the covering film (14') of the blister (8) and then the inner peripheral surface of the inlet section (19) of the suction tube (S) pushes the cut film flap into the blister (8).

20. The inhaler according to any of Claims 17 to 19, wherein the cutter (20) comprises a plurality of axially extending blades (28), each having cutting edges, separated by axially-extending gaps (26).

21. The inhaler according to any of Claims 1 to 20, wherein the inlet section (19) of the suction tube (S) includes at least one passageway (29) for providing fluid communication between a blister (8) of the blister pack (B) and a space above the blister (8) when the inlet section (19) of the suction tube (S) is inserted in the blister (8).

22. The inhaler according to Claim 21, wherein the at least one passageway (29) comprises an open channel in the outer peripheral surface of the inlet section (19) of the suction tube (S), the at least one passageway (29) having one end located at a position along the length of the inlet section (19) of the suction tube (S) and the other end located adjacent the inlet of the suction tube (S).

23. The inhaler according to Claim 22 when appendant upon any of Claims 17 to 20, wherein the other end of the at least one passageway (29) is located adjacent the cutter (20).

24. The inhaler according to Claim 22 or 23 when appendant upon Claim 20, wherein the other end of at least one passageway (29) is located at one of the gaps (26).

25. The inhaler according to Claim 22 or 23 when appendant upon Claim 20, wherein the other end of the at least one passageway (29) is located between adjacent gaps (26).

26. The inhaler according to Claim 21, wherein the at least one passageway (29) comprises a conduit in the inlet section (19) of the suction tube (S), the at least one passageway (29) having an inlet (29') located at a position along the length of the inlet section (19) of the auction tube (S) and an outlet (29") located adjacent the inlet of the suction tube (S).

27. The inhaler according to Claim 26, when appendant upon any of Claims 17 to 20, wherein the outlet (29") of the at least one passageway (29) is located adjacent the cutter (20).

28. The inhaler according to Claim 26 or 27 when appendant upon Claim 20, wherein the outlet (29") of the at least one passageway (29) is located at one of the gaps (26).

29. The inhaler according to Claim 26 or 27 when appendant upon Claim 20, wherein the outlet (29") of the at least one passageway (29) is located between adjacent gaps (26).

30. The inhaler according to any of Claims 1 to 29, wherein the suction tube (S) includes at least one passageway (32) providing fluid communication between the inhalation channel (31) and the atmosphere through which supplementary air is in use drawn into the inhalation channel (31) on inhalation by the user.

31. The inhaler according to any of Claims 1 to 30, wherein the inlet section (19) of the suction tube (S) includes a radially -extending member defining a shoulder (22) at a predetermined axial distance from the distal end thereof, the shoulder (22) being configured so as in use to abut the blister pack (B) and prevent the inlet section (19) of the suction tube (S) from being inserted too far into a blister (8) of the blister pack (B).

32. The inhaler according to any of Claims 1 to 31, further comprising a member connecting the suction tube (S) to the support unit (V,G) to prevent the suction tube (S) and the support unit (V,G) from being separated from one another.

## Patentansprüche

1. Inhalator für die Verabreichung von Trockenpulver in Kombination mit einer Blisterpackung (B), wobei der Inhalator ein Saugrohr (S) mit einem Einlass an einem Ende und einem Auslass am anderen Ende umfasst, wobei das Saugrohr (S) an einem Ende einen Einlassabschnitt (19) aufweist, der so konfiguriert ist, dass er in die eine Vielzahl von Blistern (8) mit je einer Arzneimittel (9) enthaltenden Pulverdosis aufweisende Blisterpackung (B) eingeführt werden kann, und einen Inhalationskanal (31), der für die Fluidverbindung zwischen dem Einlass und dem Auslass sorgt und durch den das Pulver beim Gebrauch durch Inhalation von einem Anwender hindurchgezogen wird, **dadurch gekennzeichnet, dass** der Inhalator ferner eine Stützeinheit (V, G) umfasst, die so konfiguriert ist, dass sie die Blisterpackung (B) stützt, wobei die Stützeinheit (V, G) eine Führungswand (6) aufweist, neben der die Blisterpackung (B) beim Gebrauch angeordnet ist, wobei die Führungswand (6) eine Vielzahl von Öffnungen (15) einschließt, die als zu den jeweiligen Blistern (8) der Blisterpackung (B) ausgerichtete Führungsteile wirken, wobei jede Öffnung (15) zum Führen des Einlassabschnittes (19) des Saugrohres (S) in einen jeweiligen Blister (8) der Blisterpackung (B) und zum Unterstützen des Saugrohrs (S) während dieser Führung dient.

2. Inhalator nach Anspruch 1, bei dem die Stützeinheit (V) ein Behälter ist, in dem die Blisterpackung (B) beim Gebrauch untergebracht ist.

3. Inhalator nach Anspruch 2, bei dem die Stützeinheit (V) ein Stützglied (3) mit einer Vielzahl von Hohlräumen (12) zur Aufnahme jeweiliger Blister (8) der Blisterpackung (B) einschließt, wobei die Hohlräume (12) genau so angeordnet sind wie die Öffnungen (15) in der Führungswand (6), so dass die Blister (8) der Blisterpackung (B) beim Gebrauch den jeweiligen Öffnungen (15) gegenüberliegen.

4. Inhalator nach Anspruch 3, bei dem das Stützglied (3) zum Beladen und Entladen der Blisterpackungen (B) relativ zur Führungswand (6) verschiebbar ist.

5. Inhalator nach Anspruch 1, bei dem die Stützeinheit (G) ein Haltemittel zum Halten der im Wesentlichen planaren Fläche der Blisterpackung (B) neben der Führungswand (6) einschließt.

6. Inhalator nach Anspruch 5, bei dem das Haltemittel C-förmige Lippen zur verschiebbaren Aufnahme der Blisterpackung (B) umfasst.

7. Inhalator nach Anspruch 5 oder 6, bei dem die Öffnungen (15) durch längliche Abschnitte (36) definiert werden.

8. Inhalator nach Anspruch 7, bei dem die Länge der länglichen Abschnitte (36) größer ist als die Breite der Blister (8) der Blisterpackung (B).

9. Inhalator nach einem der Ansprüche 1 bis 8, bei dem die Stützeinheit (V, G) einen oder mehrere Vorsprünge oder Vertiefungen (41) einer solchen vorbestimmten Größe und Position einschließt, dass die Stützeinheit (V, G) nur Blisterpackungen (B) mit entsprechenden Vertiefungen oder Vorsprüngen (40) aufnehmen kann.

10. Inhalator nach Anspruch 9, bei dem der eine Vorsprung oder die eine Vertiefung bzw. die mehreren Vorsprünge oder Vertiefungen (41) eine solche Größe aufweisen und so positioniert sind, dass die Stützeinheit (V, G) nur Blisterpackungen (B) in einer relativen Orientierung aufnehmen kann.

11. Inhalator nach einem der Ansprüche 1 bis 10, bei dem der Einlassabschnitt (19) des Saugrohres (S) und jede der Öffnungen (15) so konfiguriert sind, dass der Einlassabschnitt (19) des Saugrohres (S) nur in einer relativen Orientierung durch die Öffnungen (15) eingeführt werden kann.

12. Inhalator nach Anspruch 11, bei dem der Querschnitt des Einlassabschnittes (19) des Saugrohres (S) und der Querschnitt jeder der Öffnungen (15) rotationsasymmetrisch sind.

13. Inhalator nach Anspruch 12, bei dem jede der Öffnungen (15) eine im allgemeinen kreisförmige Gestalt mit mindestens einer radialen Verlängerung (16, 16', 16") aufweist.

14. Inhalator nach Anspruch 13, bei dem mehrere Öffnungen (15) durch mindestens eine radiale Verlängerung (16) miteinander verbunden sind.

15. Inhalator nach einem der Ansprüche 1 bis 14, bei dem die Stützeinheit (V, G) einen Halter zum Halten des Saugrohres (S) bei Nichtgebrauch einschließt.

16. Inhalator nach Anspruch 15, bei dem der Halter eine Kammer (34) mit einem Scharnierdeckel (33) umfasst.

17. Inhalator nach einem der Ansprüche 1 bis 16, bei dem der Einlassabschnitt (19) des Saugrohres (S) ein Schneidmesser (20) mit einer Schneidkante zum Einschneiden einer Abdeckfolie (14') eines Blisters (8) der Blisterpackung (B) einschließt.

18. Inhalator nach Anspruch 17, bei dem das Schneidmesser (20) nur um einen Teil des Einlasses des Saugrohres (S) angeordnet ist, so dass das Schneidmesser (20) beim Einführen des Einlassabschnittes (19) des Saugrohres (S) in einen Blister (8) der Blisterpackung (B) die Abdeckfolie (14') des Blisters (8) so einschneidet, dass eine einmal eingeschnittene Folienklappe übrig bleibt.

19. Inhalator nach Anspruch 18, bei dem die Schneidkante des Schneidmessers (20) an der äußeren Umfangsfläche des Einlassabschnittes (19) des Saugrohres (S) so angeordnet ist, dass das Schneidmesser (20) beim Einführen des Einlassabschnittes (19) des Saugrohres (S) in einen Blister (8) der Blisterpackung (B) zunächst die Abdeckfolie (14') des Blisters (8) einschneidet und die innere Umfangsfläche des Einlassabschnittes (19) des Saugrohres (S) dann die eingeschnittene Folienklappe in den Blister (8) drückt.

20. Inhalator nach einem der Ansprüche 17 bis 19, bei dem das Schneidmesser (20) eine Vielzahl von sich axial erstreckenden Klingen (28) umfasst, die jeweils Schneidkanten aufweisen und durch sich axial erstreckende Lücken (26) voneinander getrennt sind.

21. Inhalator nach einem der Ansprüche 1 bis 20, bei dem der Einlassabschnitt (19) des Saugrohres (S) mindestens einen Durchgang (29) für die Fluidverbindung zwischen einem Blister (8) der Blisterpackung (B) und einem Raum über dem Blister (8), wenn der Einlassabschnitt (19) des Saugrohres (S) in den Blister (8) eingeführt ist, einschließt.

22. Inhalator nach Anspruch 21, bei dem der mindestens eine Durchgang (29) einen offenen Kanal in der äußeren Umfangsfläche des Einlassabschnittes (19) des Saugrohres (S) umfasst, wobei ein Ende des mindestens einen Durchgangs (29) an einer Stelle entlang der Länge des Einlassabschnittes (19) des Saugrohres (S) und das andere Ende neben dem Einlass des Saugrohres (S) angeordnet ist.

23. Inhalator nach Anspruch 22 in Abhängigkeit von einem der Ansprüche 17 bis 20, bei dem das andere Ende des mindestens einen Durchgangs (29) neben dem Schneidmesser (20) angeordnet ist.

24. Inhalator nach Anspruch 22 oder 23 in Abhängigkeit von Anspruch 20, bei dem das andere Ende des mindestens einen Durchgangs (29) an einer der Lücken (26) angeordnet ist.

25. Inhalator nach Anspruch 22 oder 23 in Abhängigkeit von Anspruch 20, bei dem das andere Ende des mindestens einen Durchgangs (29) zwischen benachbarten Lücken (26) angeordnet ist.

26. Inhalator nach Anspruch 21, bei dem der mindestens eine Durchgang (29) eine Leitung im Einlassabschnitt (19) des Saugrohres (S) umfasst, wobei der mindestens eine Durchgang (29) einen Einlass (29') an einer Stelle entlang der Länge des Einlassabschnittes (19) des Saugrohres (S) und einen Auslass (29") neben dem Einlass des Saugrohres (S) aufweist.

27. Inhalator nach Anspruch 26 in Abhängigkeit von einem der Ansprüche 17 bis 20, bei dem der Auslass (29") des mindestens einen Durchgangs (29) neben dem Schneidmesser (20) angeordnet ist.

28. Inhalator nach Anspruch 26 oder 27 in Abhängigkeit von Anspruch 20, bei dem der Auslass (29") des mindestens einen Durchgangs (29) an einer der Lücken (26) angeordnet ist.

29. Inhalator nach Anspruch 26 oder 27 in Abhängigkeit von Anspruch 20, bei dem der Auslass (29") des mindestens einen Durchgangs (29) zwischen benachbarten Lücken (26) angeordnet ist.

30. Inhalator nach einem der Ansprüche 1 bis 29, bei dem das Saugrohr (S) mindestens einen Durchgang (29) für die Fluidverbindung zwischen dem Inhalationskanal (31) und der Atmosphäre einschließt, durch den beim Gebrauch durch Inhalation von dem Anwender zusätzliche Luft in den Inhalationskanal (31) gezogen wird.

31. Inhalator nach einem der Ansprüche 1 bis 30, bei dem der Einlassabschnitt (19) des Saugrohres (S) ein sich radial erstreckendes Glied einschließt, das eine Schulter (22) in einem vorbestimmten axialen Abstand vom distalen Ende davon definiert, wobei die Schulter (22) so konfiguriert ist, dass sie beim Gebrauch an der Blisterpackung (B) anliegt und verhindert, dass der Einlassabschnitt (19) des Saugrohres (S) zu weit in den Blister (8) der Blisterpackung (B) eingeführt wird.

32. Inhalator nach einem der Ansprüche 1 bis 31, weiterhin umfassend ein Glied, das das Saugrohr (S) mit der Stützeinheit (G, V) verbindet, um zu verhindern, dass das Saugrohr (S) und die Stützeinheit (V, G) voneinander getrennt werden.

## Revendications

1. Inhalateur pour l'administration d'une poudre sèche, en combinaison avec un emballage pelliculé (B), l'inhalateur comprenant un tube d'aspiration (S) ayant une entrée à une extrémité et une sortie à l'autre extrémité, le tube d'aspiration (S) comprenant, à la première extrémité, une section d'entrée (19) configurée pour une insertion dans l'emballage pelliculé (B) ayant une pluralité de bulles (8) contenant chacune une dose de poudre contenant un médicament (9), et un canal d'inhalation (31) établissant une communication de fluide entre l'entrée et la sortie, par lequel la poudre est, en service, aspirée par l'inhalation d'un utilisateur, **caractérisé en ce que** l'inhalateur comprend par ailleurs une unité de support (V,G) configurée pour supporter l'emballage pelliculé (B), l'unité de support (V,G) comprenant une paroi de guidage (6) près de laquelle l'emballage pelliculé (B) est disposé en service, la paroi de guidage (6) comprenant une pluralité d'ouvertures (15) agissant comme des parties de guidage dans l'alignement avec des bulles respectives (8) de l'emballage pelliculé (B), chaque ouverture (15) servant à guider la section d'entrée (19) du tube d'aspiration (S) dans une bulle respective (8) de l'emballage pelliculé (B) et supportant le tube d'aspiration (S) lorsqu'il est ainsi guidé.

2. Inhalateur selon la revendication 1, dans lequel l'unité de support (V) est un récipient dans lequel est logé l'emballage pelliculé (B) utilisé.

3. Inhalateur selon la revendication 2, dans lequel l'unité de support (V) comprend un élément de support (3) comprenant une pluralité de cavités (12) pour recevoir des bulles respectives (8) de l'emballage pelliculé (B), les cavités (12) ayant le même aménagement que les ouvertures (15) dans la paroi de guidage (6) de sorte que, en service, les bulles (8) de l'emballage pelliculé (B) fassent face aux ouvertures respectives (15).

4. Inhalateur selon la revendication 3, dans lequel l'élément de support (3) est à même de glisser par rapport à la paroi de guidage (6) de manière à permettre le chargement et le déchargement d'emballages pelliculés (B).

5. Inhalateur selon la revendication 1, dans lequel l'unité de support (G) comprend des moyens de retenue pour retenir la surface sensiblement plane de l'emballage pelliculé (B) adjacente à la paroi de guidage (6).

6. Inhalateur selon la revendication 5, dans lequel le moyen de retenue comprend des lèvres en forme de C pour recevoir par glissement l'emballage pelliculé (B).

7. Inhalateur selon la revendication 5 ou 6, dans lequel les ouvertures (15) sont définies par des sections allongées (36).

8. Inhalateur selon la revendication 7, dans lequel la longueur des sections allongées (36) est supérieure à la largeur des bulles (8) de l'emballage pelliculé (B).

9. Inhalateur selon l'une quelconque des revendications 1 à 8, dans lequel l'unité de support (V,G) comprend une ou plusieurs saillies ou cavités (41) de taille et de position prédéterminées de sorte que l'unité de support (V,G) ne puisse recevoir que des emballages pelliculés (B) présentant des cavités ou des saillies correspondantes (40).

10. Inhalateur selon la revendication 9, dans lequel la ou les saillies ou cavités (41) sont dimensionnées et positionnées de sorte que l'unité de support (V,G) ne puisse recevoir que des emballages pelliculés (B) disposés dans une seule orientation relative.

11. Inhalateur selon l'une quelconque des revendications 1 à 10, dans lequel la section d'entrée (19) du tube d'aspiration (S) et chacune des ouvertures (15) sont configurées de sorte que la section d'entrée (19) du tube d'aspiration (S) ne puisse être insérée qu'à travers les ouvertures (15) ménagées dans une seule orientation relative.

12. Inhalateur selon la revendication 11, dans lequel la section transversale de la section d'entrée (19) du tube d'aspiration (S) et la section transversale de chacune des ouvertures (15) sont asymétriques en rotation.

13. Inhalateur selon la revendication 12, dans lequel chacune des ouvertures (15) est de forme généralement circulaire avec au moins une extension radiale (16,16',16").

14. Inhalateur selon la revendication 13, dans lequel une pluralité des ouvertures (15) sont raccordées par au moins une extension radiale (16).

15. Inhalateur selon l'une quelconque des revendications 1 à 14, dans lequel l'unité de support (V,G) comprend un élément de retenue pour retenir le tube d'aspiration (S) lorsqu'il n'est pas utilisé.

16. Inhalateur selon la revendication 15, dans lequel l'élément de retenue comprend une chambre (34) avec un couvercle à charnière (33).

17. Inhalateur selon l'une quelconque des revendications 1 à 16, dans lequel la section d'entrée (19) du tube d'aspiration (S) comprend un couteau (20) ayant une arête tranchante pour couper une pellicule de recouvrement (14') d'une bulle (8) de l'emballage pelliculé (B).

18. Inhalateur selon la revendication 17, dans lequel le couteau (20) est situé autour d'une partie seulement de l'entrée du tube d'aspiration (S) de sorte que, lors de l'insertion de la section d'entrée (19) du tube d'aspiration (S) dans une bulle (8) de l'emballage pelliculé (B), le couteau (20) coupe la pellicule de recouvrement (14') de la bulle (8) de manière à laisser un simple rabat de pellicule coupée.

19. Inhalateur selon la revendication 18, dans lequel l'arête tranchante du couteau (20) est disposée sur la surface périphérique externe de la section d'entrée (19) du tube d'aspiration (S) de sorte que, lorsque la section d'entrée (19) du tube d'aspiration (S) est insérée dans une bulle (8) de l'emballage pelliculé (B), le couteau (20) coupe d'abord la pellicule de recouvrement (14') de la bulle (8) et ensuite la surface périphérique interne de la section d'entrée (19) du tube d'aspiration (S) pousse le rabat de pellicule coupée dans la bulle (8).

20. Inhalateur selon l'une quelconque des revendications 17 à 19, dans lequel le couteau (20) comprend une pluralité de lames (28) s'étendant axialement, chacune ayant des arêtes tranchantes, séparées par des intervalles (26) qui s'étendent axialement.

21. Inhalateur selon l'une quelconque des revendications 1 à 20, dans lequel la section d'entrée (19) du tube d'aspiration (S) comprend au moins un passage (29) pour établir une communication de fluide entre une bulle (8) de l'emballage pelliculé (B) et un espace au-dessus de la bulle (8) lorsque la section d'entrée (19) du tube d'aspiration (S) est insérée dans la bulle (8).

22. Inhalateur selon la revendication 21, dans lequel le au moins un passage (29) comprend un canal ouvert dans la surface périphérique externe de la section d'entrée (19) du tube d'aspiration (S), le au moins un passage (29) ayant une extrémité située dans une position sur la longueur de la section d'entrée (19) du tube d'aspiration (S) et l'autre extrémité située adjacente à l'entrée du tube d'aspiration (S).

23. Inhalateur selon la revendication 22, lorsqu'elle est apparentée à l'une quelconque des revendications 17 à 20, dans lequel l'autre extrémité du au moins un passage (29) est disposée adjacente au couteau (20).

24. Inhalateur selon la revendication 22 ou 23, lorsqu'elle est apparentée à la revendication 20, dans lequel l'autre extrémité du au moins un passage (29) est située dans l'un des intervalles (26).

25. Inhalateur selon la revendication 22 ou 23, lorsqu'elle est apparentée à la revendication 20, dans lequel l'autre extrémité du au moins un passage (29) est située entre des intervalles adjacents (26).

26. Inhalateur selon la revendication 21, dans lequel le au moins un passage (29) comprend une conduite dans la section d'entrée (19) du tube d'aspiration (S), le au moins un passage (29) ayant une entrée (29') située dans une position sur la longueur de la section d'entrée (19) du tube d'aspiration (S) et une sortie (29") située adjacente à l'entrée du tube d'aspiration (S).

27. Inhalateur selon la revendication 26, lorsqu'elle est apparentée à l'une quelconque des revendications 17 à 20, dans lequel la sortie (29") du au moins un passage (29) est située adjacente au couteau (20).

28. Inhalateur selon la revendication 26 ou 27, lorsqu'elle est apparentée à la revendication 20, dans lequel la sortie (29") du au moins un passage (29) est située dans l'un des intervalles (26).

29. Inhalateur selon la revendication 26 ou 27, lorsqu'elle est apparentée à la revendication 20, dans lequel la sortie (29") du au moins un passage (29) est située entre des intervalles adjacents (26).

30. Inhalateur selon l'une quelconque des revendications 1 à 29, dans lequel le tube d'aspiration (S) comprend au moins un passage (32) établissant une communication de fluide entre le canal d'inhalation (31) et l'atmosphère, à travers lequel de l'air supplémentaire est, en service, aspiré dans le canal d'inhalation (31) lors d'une inhalation par l'utilisateur.

31. Inhalateur selon l'une quelconque des revendications 1 à 30, dans lequel la section d'entrée (19) du tube d'aspiration (S) comprend un élément s'étendant radialement définissant un épaulement (22) à une distance axiale prédéterminée de son extrémité distale, l'épaulement (22) étant configuré de sorte que, en service, il s'appuie sur l'emballage pelliculé (B) et empêche la section d'entrée (19) du tube d'aspiration (S) d'être insérée trop loin dans une bulle (8) de l'emballage pelliculé (B).

32. Inhalateur selon l'une quelconque des revendications 1 à 31, comprenant par ailleurs un élément raccordant le tube d'aspiration (S) à l'unité de support (V,G) pour empêcher la séparation du tube d'aspiration (S) et de l'unité de support (V,G) l'un de l'autre.
